(19) Europäisches Patentamt / European Patent Office / Office européen des brevets

(11) **EP 2 864 390 B1**

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(21) Anmeldenummer: **13734355.4**

(22) Anmeldetag: **19.06.2013**

(51) Int Cl.:
**C08G 63/52** (2006.01)    **C08G 63/676** (2006.01)
**C09D 167/06** (2006.01)    **C08L 67/06** (2006.01)

(86) Internationale Anmeldenummer:
**PCT/EP2013/062776**

(87) Internationale Veröffentlichungsnummer:
**WO 2013/190000 (27.12.2013 Gazette 2013/52)**

(54) **HOCHZÄHE WERKSTOFFE AUF BASIS UNGESÄTTIGTER POLYESTER**

HIGH-TOUGHNESS MATERIALS BASED ON UNSATURATED POLYESTERS

MATIÈRES À HAUTE TÉNACITÉ À BASE DE POLYESTER INSATURÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.06.2012 DE 102012210483**
**15.10.2012 DE 102012109803**

(43) Veröffentlichungstag der Anmeldung:
**29.04.2015 Patentblatt 2015/18**

(73) Patentinhaber: **Fraunhofer-Gesellschaft zur Förderung der angewandten Forschung e.V.
80686 München (DE)**

(72) Erfinder:
• **LORENZ, Reinhard**
  **Steinfurt 48565 (DE)**
• **BAUER, Monika**
  **15712 Königs-Wusterhausen (DE)**
• **STEFFEN, Sebastian**
  **12163 Berlin (DE)**

(74) Vertreter: **Strehl Schübel-Hopf & Partner Maximilianstrasse 54
80538 München (DE)**

(56) Entgegenhaltungen:
**DE-A1- 2 706 308    DE-T2- 69 924 863**
**GB-A- 722 265**

• **DATABASE CA [Online] CHEMICAL ABSTRACTS SERVICE, COLUMBUS, OHIO, US; MIKHAILOVA, Z. V. ET AL: "Effect of structure on the physical and mechanical properties of chemical-resistant unsaturated polyester resins", XP002714087, gefunden im STN Database accession no. 1980:199205 in der Anmeldung erwähnt & MIKHAILOVA, Z. V. ET AL: "Effect of structure on the physical and mechanical properties of chemical-resistant unsaturated polyester resins", POLIMERY (WARSAW, POLAND) , 24(11-12), 407-9 CODEN: POLIA4; ISSN: 0032-2725, 1979,**

Bemerkungen:
Die Akte enthält technische Angaben, die nach dem Eingang der Anmeldung eingereicht wurden und die nicht in dieser Patentschrift enthalten sind.

EP 2 864 390 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ungesättigte Polyesterharze mit hoher Risszähigkeit, umfassend ein Weichdiolsegment sowie ein Hartdiolsegment.

Ungesättigte Polyesterharze werden in der technischen Praxis in großem Umfang faserverstärkt eingesetzt. Typische Einsatzgebiete sind der Bootsbau, der Bau von Tanks, Behältern, Rohrleitungen und Wäschern, das Relining (Kanalsanierung), die Herstellung Platten sowie von Pressteilen nach dem SMC-Verfahren oder von Spritzgussteilen nach dem BMC-Verfahren. Mit dem SMC- und BMC-Verfahren werden Bauteile für die Elektroindustrie (zum Beispiel Langfeldleuchtenträger, Schaltkästen, Kommutatoren), für die Bauindustrie (zum Beispiel Kellerfensterschächte, Rohrelemente und andere Bauelemente) oder für den Fahrzeugbau (zum Beispiel Karosserieteile für PKW und LKW, Scheinwerferreflektoren, Bauteile für die Innenausstattung von Eisenbahnwaggons) erzeugt. Im begrenzten Umfang werden ungesättigte Polyesterharze auch für die Herstellung von Rotorblättern von Windkraftanlagen verwendet. In dieser Anwendung konkurrieren sie mit Epoxidharzen, die eine deutlich höhere Risszähigkeit aufweisen und damit eine 'schlankere', massenärmere Bauweise ermöglichen, da sie weniger steif ausgelegt werden müssen und größere Verformungen zugelassen werden können, wodurch längere Rotorblätter (z.B. für Offshore-Anwendungen) ermöglicht werden.

Unverstärkte Anwendungen ungesättigter Polyesterharze finden sich zum Beispiel bei Agglomarmor für Sanitärartikel oder Möbel, bei Papierlaminaten für die Möbelbeschichtung, beim Polymerbeton, bei Knöpfen für Hemden und andere Bekleidungsartikel, bei Spachtel- und Reparaturmassen. Zudem sind die diversen Beschichtungsanwendungen zu nennen, die unter anderem unter den Bezeichnungen Gelcoat, Topcoat, Inmouldcoat, Lack oder Korrosionsschutzauskleidung zur Anwendung kommen und die in verschiedener Weise der Oberflächenveredelung bzw. dem Oberflächenschutz dienen.

Eine erste Charakterisierung der Risszähigkeit ist bruchmechanisch über den sogenannten $k_{1C}$-Wert (auch als Spannungsintensitätsfaktor im Verformungsmodus I bezeichnet) möglich. Bei konventionellen marktüblichen UP-Harzen werden $k_{1C}$-Werte zwischen 0,35 und etwa 0,5 gemessen, bei Epoxidharzen für die genannten Windkraftanwendungen sind Werte von etwa 0,8 üblich. Alternativ zu ungesättigten Polyestern und Epoxidharzen werden auch die Kombinationsklassen der Vinylester- und Vinylesterurethanharze (VE- bzw. VEU-Harze) in der Windkraft eingesetzt, die $k_{1C}$-Werte zwischen etwa 0,5 und 0,8 zeigen.

DE 699 24 863 T2 offenbart flexible ungesättigte Polyesterharze, die bei Temperaturen unterhalb von 0°C eingesetzt werden können und sich durch eine sehr hohe Bewitterungsbeständigkeit auszeichnen. Diese Harze besitzen Molekulargewichte zwischen 20.000 und 100.000 und sind unter Verwendung von (Poly-)Glykolverbindungen mit Molekulargewichten zwischen 300 und 12.000 und mit einer Gesamthydroxyfunktionalität von 2-4 pro Molekül hergestellt. Bevorzugt ist ein Verhältnis von 60-80 Mol-% Diolen zu 20-40-Mol-% Triolen.

In der Veröffentlichung von S. B. Pandit und V. M. Nadkarni "Toughening of unsaturated Polyesters by Reactive Liquid Polymers. 1. Synthesis and Characterization of the Modifiers", in Ind. Eng. Chem. Res. 1993, 32, 3089-3099 und "Toughening of unsaturated Polyesters by Reactive Liquid Polymers. 2. Processibility and mechanical Properties", in Ind. Eng. Chem. Res 1994, 33, 2778-2788 derselben Autoren werden Zähigkeitsmodifikatoren auf Basis eines ungesättigten Polyesters aus 1 mol MSA und 2 mol PPG oder PEG miteinander verglichen.

DE 44 06 646 A1 beschreibt Produkte aus trifunktionellen Polypropylenoxiden, die endständig mit MSA zu sogenannten Halbestern umgesetzt werden, ohne dass eine weitere Polykondensation erfolgt. Diese Produkte werden in Vinylester- und Vinylesterurethanharzen als sogenannte Eindickadditive für die MgO-basierte Eindickung eingesetzt, wobei sie die für die Eindickung erforderlichen COOH-Gruppen bereitstellen, die der VE bzw. VU nicht aufweisen.

In dem CAPLUS-Abstract 1980:199205 zu einem Artikel von Mikhaiolova, Z.V. et al. In Polimery (Warsaw, Poland) (1979), 24(11-12), 407-409 wird darauf hingewiesen, dass die Einarbeitung von Polypropylenglycol in die Ketten ungesättiger Polyester aus propoxyliertem Bisphenol A und Maleinanhydrid zu einer verbesserten Elastizität des daraus erhältlichen, styrolvernetzten Produkts führt, dass aber über den untersuchten Molekulargewichts-Bereich des eingesetzten Polypropylenglycols hinweg (500 bis 4000) mit steigendem Molekulargewicht die chemische Widerstandsfähigkeit des styrolvernetzten Produktes sinkt. Ob 1,2- oder 1,3-Polypropylenglycol oder eine Mischung aus beidem eingesetzt wurde, lässt sich dem Abstract nicht entnehmen.

GB 722 265 offenbart einen ungesättigten Carbonsäureester, der aus der Umsetzung von Malein- oder Fumarsäure mit einem Diolgemisch erhältlich ist. Letzteres besteht zu 50 bis 90 Mol-% aus einem dialkoxylierten Bisphenol A und zu 10 bis 50 Mol-% aus mindestens einem weiteren Diol.

Ungesättigte Polyesterharze weisen nach der Vernetzung mit einem Reaktivverdünner eine breite Verteilung der Molmasse und Länge der Netzwerkketten auf (gemeint ist der Netzbogen zwischen zwei Vernetzungspunkten). Dies ergibt sich aus der statistischen Natur der eingesetzten ungesättigten Polyester, die in der Regel zwei Dicarbonsäuren aufweisen, eine vernetzungsfähige Dicarbonsäure (meist Malein- und Fumarsäureester) und eine nichtvernetzungsfähige Dicarbonsäure (z.B. Phthalsäure, Isophthalsäure oder Adipinsäure). Zudem ist die Netzbogenlänge der radikalisch gebildeten Kette (im vernetzten Produkt) sehr kurz, da sich zwischen zwei Malein- bzw. Fumarestereeinheiten im Mittel nur zwei bis drei Einheiten Reaktivverdünner (in der Regel Styrol) befinden. Insgesamt erhält man somit bei ungesättigten Polyestern sehr heterogene Netzwerke, in denen engmaschig vernetzte Abschnitte neben deutlich weniger engmaschigen Abschnitten vorliegen. Die Glastemperatur der vernetzten ungesättigten Polyesterharze wird im Wesentlichen über das Verhältnis von vernetzungsfähiger zu nicht vernetzungsfähiger Dicarbonsäure gesteuert; dieses Konzept dominiert in der Chemie der ungesättigten Polyester. Daneben ist es üblich, ungesättigte Polyester mit hoher Vernetzungsdichte mit solchen mit niedriger Vernetzungsdichte zu mischen, um ein größeres Intervall bei der Wärmeformbeständigkeit und der Glastemperatur abzudecken. Dabei gilt generell der Zusammenhang je höher die Glastemperatur, desto geringer die Werkstoffzähigkeit. Es ist verständlich, dass bei diesem konventionellen Konzept der UP-Harz-Technologie stets Bereiche mit hoher Vernetzungsdichte gebildet werden, die die Zähigkeit der UP-Harz-basierten Werkstoffe stark begrenzen.

Mit der Erfindung wird vorgeschlagen, diesen Nachteil zu umgehen. Dies gelingt in überraschender Weise durch einen grundlegend neuen Ansatz, bei dem die chemische Architektur der ungesättigten Polyester so modifiziert wird, dass ein möglichst gleichmäßiger Abstand der Vernetzungspunkte voneinander erzielt wird. Dabei kann die Glastemperatur auf einem für UP-Harze neuen Wege eingestellt werden, nämlich durch die Kombination zweier Diole vorzugsweise etwa gleicher Kettenlänge, die sich in ihrer Kettensteifigkeit und damit in ihrem Beitrag zur Glastemperatur deutlich unterscheiden. Hierfür wird zum Beispiel die Stoffklasse der diethoxylierten oder dipropoxylierten Bisphenole - im Folgenden als Hartdiolsegment bezeichnet - sowie die Stoffklasse der Propylenoxide bzw. Polypropylenglykole sowie der Ethylenoxid-Propylenoxidcoglykole - im Folgendem als Weichdiolsegment bezeichnet - vorgeschlagen. Über das Mischungsverhältnis von Weich- zu Hartdiolsegment kann die Glastemperatur und die Zähigkeit des Netzwerks in weiten Grenzen eingestellt werden.

Erfindungsgemäß werden demnach ungesättigte Polyesterharze gemäß Anspruch 1 aus oder mit den nachfolgend in Formel (I) definierten Ausgangsmaterialien bereitgestellt:

$$A_{(0,9-1,2)} \, (B + C)_{(1,0)} \, , \qquad (I)$$

wobei die molaren Mengenverhältnisse von Ausgangsmaterial A zur Summe der Ausgangsmaterialien B und C in Klammern angegeben sind.

Das Ausgangsmaterial A in dieser Formel ist eine ungesättigte Dicarbonsäure, die ausgewählt werden kann unter einer oder mehreren Carbonsäuren und/oder deren Anhydriden. Der Fachmann auf dem Gebiet der Synthese ungesättigter Polyesterharze kennt die hierfür geeigneten Ausgangsmaterialien. Bevorzugt sind Fumarsäure, Maleinsäure, Itaconsäure und dergleichen, sowie die entsprechenden Anhydride.

Das Ausgangsmaterial B wird als Hartdiolsegment bezeichnet. Es besitzt (mindestens) zwei Hydroxygruppen und eine Molekülstruktur mit einer durchgehenden Kettenlänge von 5 bis 30, vorzugsweise 5 bis 25 und stärker bevorzugt 10 bis 25 Atomen zwischen den beiden Hydroxygruppen (oder zwischen jeweils zwei der Hydroxygruppen, falls mehr als zwei Hydroxygruppen vorhanden sein sollten), wobei diese Atome wie folgt berechnet werden: die endständigen Hydroxygruppen werden nicht mitgezählt; zur Kette zählen jedoch alle zwischen diesen Hydroxygruppen befindlichen Kettenglieder, d.h. nicht nur die Kohlenstoffatome, sondern auch N-, O- und S-Atome, sofern vorhanden. Substituenten bzw. Atome aus abzweigenden Gruppierungen zählen nicht mit. Sofern Ringstrukturen in der Kette vorliegen, zählen nur diejenigen Atome der Ringstruktur, die zur Kettenverlängerung beitragen. In diesem Fall wird der kürzeste Abstand zwischen den OH-Gruppen für die Berechnung der Kettenlänge gewählt. Beispielsweise sind das bei einem p-Phenylenrest 4 Kohlenstoffatome, bei einem m-Phenylenrest 3 Kohlenstoffatome und bei einem o-Phenylenrest 2 Kohlenstoffatome. Wenn als Hardiolsegment eine Mischung von zwei oder mehr Substanzen eingesetzt wird, müssen die vorgenannten Bedingungen auf die Mischung zutreffen. Das bedeutet, dass nicht jede der Substanzen als solche diesen Bedingungen gehorchen muss, auch wenn dies von Vorteil ist, sofern die Kettenlänge im Durchschnitt, bezogen auf die molaren Anteile der jeweiligen Substanzen am Ausgangsmaterial B, im obengenannten Bereich liegt. Es ist dabei natürlich günstig, wenn die Kettenlängen in einer solchen Mischung gar nicht oder nur wenig, z.B. im Bereich von ≤ 20%, voneinander abweichen.

Das Hartdiolsegment B besitzt einen Anteil von mindestens 40%, vorzugsweise mindestens 50% und besonders bevorzugt von mindestens 70% Atomen, die einer kettensteifen Gruppierung angehören, bezogen auf die Summe der C-, N-, O- und S-Atome des Moleküls (d.h. nicht nur auf die Atome der Kette bezogen), mit Ausnahme der Sauerstoffatome der endständigen Hydroxygruppen. Als kettensteife Gruppierungen werden in der Literatur solche Gruppierungen bezeichnet, bei denen Monomereinheiten so verknüpft sind, dass die gesamte Gruppierung starr ist in dem Sinne, dass sie annähernd oder im Wesentlichen eine (lineare) Stäbchenstruktur aufweist. Dies wird dadurch erreicht, dass die dem Rückgrat des Moleküls in diesem Bereich angehörenden Atome so miteinander verbunden sind, dass eine Rotation zweier benachbarter Atome entweder aus chemischen Gründen (z.B. bei einem Kohlenstoffatom mit drei Bindungspartnern) oder aus sterischen Gründen unterbunden ist, oder dass die Symmetrie der Gruppierung derart ist, dass Rotation(en) um eine Einfachbindung nicht zu einer Veränderung der Achsrichtung der Gruppierung führt, wie es z.B. bei in das Rückgrat eingebetteten, planaren Ringstrukturen der Fall ist, deren Mittelachse in der Achse der entsprechenden Stäbchenstruktur liegt und auch bei Rotation um z.B. Einfachbindungen zu benachbarten Kohlenstoffatomen in dieser Achse verbleibt. Eine gute Darstellung des Konzepts der Kettensteifigkeit findet sich bei M. Ballauf in Angewandte Chemie 101(3), 362-410 (1989). Es ist dabei darauf hinzuweisen, dass dieses Konzept nicht nur auf ganze Moleküle anwendbar ist, sondern auch auf Teile davon sowie auf einzelne Gruppierungen/Gruppen, die am Ort ihres Einbaus aus den genannten Gründen zu einer Versteifung des Moleküls beitragen. Als kettensteife Gruppen für das erfindungsgemäß einsetzbare Hardiolsegment eignen sich unter Berücksichtigung der voranstehenden Erwägungen insbesondere aromatische, aliphatische und heterocyclische Ringe einschließlich mono-, bi-, tri- und polycyclischer Verbindungen, Amid-, Biuret-, Triazin-, Harnstoff-, Thioharnstoff-, Urethan- und Thiourethangruppen, para-, ortho- und metadisubstituierte $C_6$-Aromaten, Naphthylgruppen sowie Heteroaromaten. Diese Aufzählung ist nicht notwendigerweise vollständig, der Fachmann kennt weitere kettensteife Gruppierungen.

Bevorzugt als Hardiolsegment B sind dialkoxylierte, z.B. dipropoxylierte oder diethoxylierte Bisphenol A-Körper; alternativ können z.B. andere, ebenfalls alkoxylierte Bisphenol-Körper wie Bisphenol F oder Bisphenol-trimethylcyclohexan eingesetzt werden. Anhand von bis-1,2-propoxyliertem Bisphenol A sei die Berechnung der Kettenlänge und der Kettensteifigkeitgezeigt: Da die endständigen Hydroxygruppen nicht mitgezählt werden, umfasst die Kette zwei Propoxygruppen mit je zwei C-Atomen in der Kette und je einem Sauerstoffatom sowie jeweils vier C-Atome der Phenylengruppen und das C2-Atom der mittigen, 2,2-substituierten Propangruppe, so dass sich eine Kettenlänge von 15 Kohlenstoff- bzw. Sauerstoffatomen ergibt. Das Molekül besitzt zwei kettensteife, konformativ wenig bewegliche Phenylengruppen und somit 12 Atome, die einer kettensteifen Struktur zuzurechnen sind. Bei einer Gesamtzahl von 23 Kohlenstoff- und Sauerstoffatomen des Moleküls tragen 52% der Atome zur Kettensteifigkeit bei. Bemerkt sei, dass die H-Atome auf Grund ihrer geringen Masse bei dieser Betrachtung vernachlässigt werden.

Die Moleküle können neben den oben erwähnten stickstoffhaltigen kettensteifen Elementen selbstverständlich andere, z.B. sauerstoff-und schwefelhaltige Gruppierungen enthalten, und zwar insbesondere auch in der Kette selbst. Neben Ethergruppen, wie sie die alkoxylierte Verbindungen aufweisen, können das beispielsweise Estergruppen sein.

Am Beispiel von mit Glycol veresterter Terephthalsäure sei gezeigt, wo die Grenzen der Erfindung liegen: Das Molekül besitzt 12 Kettenglieder und einen Anteil von 6 kettensteifen Kohlenstoffatomen auf 16 C- und O-Atome und fällt damit nicht unter die Erfindung. Wird stattdessen mit Glycol veresterte peri-Naphthalindicarbonsäure eingesetzt, besitzt die Kette 11 Kettenglieder und einen Anteil von 12 kettensteifen Kohlenstoffatomen auf 22 Kohlenstoff- und Sauerstoffatome und lässt sich demnach erfindungsgemäß als Hartdiol-Komponente einsetzen. Solche hydroxygruppenhaltige Ester können als Präpolymere vom Polyestertyp (gesättigt) angesehen werden; in vergleichbarer Weise lassen sich Präpolymere des Polyamid- oder Polyharnstofftyps verwenden. Auch Mischungen sind möglich. Vorzugsweise trägt das Hartdiolsegment nicht mehr als zwei Hydroxygruppen.

Das Ausgangsmaterial C wird als Weichdiolsegment bezeichnet. Es handelt sich dabei im Gegensatz zum Hartdiolsegment B um ein Material mit einer flexiblen Molekülstruktur. Es besitzt wie das Hartdiolsegment B (mindestens) zwei Hydroxygruppen und eine Molekülstruktur mit einer durchgehenden Kettenlänge von 10 bis 25 Atomen zwischen den beiden Hydroxygruppen (oder zwischen jeweils zwei der Hydroxygruppen, falls mehr als zwei Hydroxygruppen vorhanden sein sollten), wobei dieselben Berechnungsregeln hierfür gelten, wie sie oben für das Hartdiolsegment dargestellt sind. Vorzugsweise enthält das Weichdiolsegment C nur zwei Hydroxygruppen. Wenn als Weichdiolsegment eine Mischung von zwei oder mehr Substanzen eingesetzt wird, müssen wie beim Hartdiolsegment die vorgenannten Bedingungen auf die Mischung zutreffen. Das bedeutet, dass nicht jede der Substanzen als solche diesen Bedingungen gehorchen muss, auch wenn dies von Vorteil ist, sofern die Kettenlänge im Durchschnitt, bezogen auf die molaren Anteile der jeweiligen Substanzen am Ausgangsmaterial C, im obengenannten Bereich liegt. Es ist dabei natürlich günstig, wenn die Kettenlängen in einer solchen Mischung gar nicht oder nur wenig, z.B. im Bereich von $\leq 20\%$, voneinander abweichen.

Das Weichdiolsegment C weist einen hohen Anteil an unverzweigten und/oder geringverzweigten Alkyl-, Polyether-, Polyester- oder Polycarbonatketten zwischen den beiden Hydroxygruppen auf, die seine Weichheit bewirken. Der Anteil an kettensteifen Strukturen, wie sie oben für das Hartdiolsegment definiert sind, darf dagegen maximal bei 25%, vorzugsweise maximal bei 20% liegen; vorzugsweise enthält das Weichdiolsegment gar keine kettensteifen Strukturen. Das Weichdiolsegment kann z.B. unter $\alpha,\omega$-Alkandiolen, Polypropylenglykolen (darunter bevorzugt Tetra- bis Heptameren mit einer Molmasse zwischen 250 bis 500, vorzugsweise bis unter 500, z.B. bis maximal ca. 450 g/mol d.h. mit nicht mehr als acht 1,2-Polypropylenglycol-Einheiten entsprechend einer Kettenlänge zwischen den Hydroxygruppen von 24), Polypropylen-Polyethylencoglykolen, Polytetrahydrofuranen, Polymeren des 6-Caprolactons, 1,6-Hexandiolcarbonaten, $CO_2$-basierten Polycarbonaten, sowie weiteren kettenweichen $\alpha,\omega$-hydroxyfunktionellen Strukturen mit einer Kettenlänge von vorzugsweise 10-24 oder 10-25 Atomen ausgewählt sein, wobei die Kette beliebig "C-analoge" Heteroatome (ausgewählt unter O, S, N) aufweisen kann, sofern diese nicht Bestandteil einer oben als kettensteif bezeichneten Struktur wie Amid-, Biuret-, Triazin-, Harnstoff-, Thioharnstoff-, Urethan- und Thiourethangruppen sind. Die letztgenannten Gruppen sind allerdings nicht ausgeschlossen, sofern sie, ggf. mit anderen Strukturen, keinen größeren Anteil als 25%, vorzugsweise als 20% wie oben definiert ausmachen.

Die (Molmengen-)Summe von B+C ist 1; das molare Verhältnis von B:C liegt in der Regel zwischen 5:95 und 95:5, bevorzugt zwischen 10:90 und 90:10, stärker bevorzugt zwischen 30:70 und 70:30 und ganz besonders bevorzugt zwischen 67:33 und 45:55. Für den Fall, dass A = 1 ist, ist die Zahl der Carbonsäure- und der Hydroxygruppen im Ausgangsmaterial in der Regel identisch (nämlich soweit alle Hart- und Weichdiolsegmente B und C jeweils genau zwei Hydroxygruppen tragen). Wenn A ungleich 1 ist, ergibt sich ein Säureunter- bzw. -überschuss, der bewusst gewählt werden und wünschenswert sein kann, um beispielsweise radikalisch reaktive Endgruppen mit Malein- und Fumarsäurehalbestern zu erhalten.

Die Kettenlänge von Hart- und Weichdiol ist im günstigsten Falle identisch; gute Ergebnisse werden jedoch auch erhalten, wenn die Anzahl der Glieder der durchgehenden Kette zwischen den beiden (bzw. bei mehr als zwei OH-Gruppen im Molekül jeweils zwischen zwei der) Hydroxygruppen im Hartdiolsegment und die Anzahl der Glieder der durchgehenden Kette zwischen den beiden (bzw. bei mehr als zwei OH-Gruppen im Molekül jeweils zwischen zwei der) Hydroxygruppen im Weichdiolsegment im Verhältnis von zwischen 1:2 und 2:1, vorzugsweise im Verhältnis von zwischen 1:1,7 bis 1,7:1 liegt.

In manchen Fällen können die Ausgangsmaterialien für die erfindungsgemäßen Harze auch eine oder mehrere gesättigte Carbonsäuren mit zwei Carboxyfunktionen aufweisen. Diese kann ausgewählt werden unter einer oder mehreren Carbonsäuren und/oder deren Anhydriden. Beispiele sind Adipinsäure oder Bernsteinsäure und deren Anhydrid. Unter dem Ausdruck "gesättigte Carbonsäure" sollen auch aromatische Säuren verstanden werden, beispielsweise Terephthalsäure, Isophthalsäure, Phthalsäure und deren Anhydride, da sie an der radikalischen Vernetzungsreaktion nicht teilnehmen. Der Fachmann auf dem Gebiet der Synthese ungesättigter Polyesterharze kennt weitere hierfür geeignete Ausgangsmaterialien. Gesättigte oder ungesättigte Monocarbonsäuren werden dagegen nur in Einzelfällen zugesetzt, z.B. für die Endgruppenverkappung, d.h. zur Verringerung endständiger OH- und COOH-Gruppen am Kettenende. Auf diesem Weg lässt sich zum Beispiel die Wasseraufnahme des gehärteten Kunststoffs graduell verringern.

Die Harzlösung kann heiß, d.h. in Schmelze des Polyesters, oder kalt im Reaktivverdünner hergestellt werden. Als Reaktivverdünner eignen sich Verbindungen mit mindestens einer C=C-Doppelbindung und ggf. einem aromatischen Ring, einem cycloaliphatischen Ring, einem Hetero-Ring, die/der unter dem Einfluss von Wärme, Licht oder ionisierender Strahlung in ein Kettenpolymer übergehen kann. Das wichtigste Beispiel hierfür ist Styrol. Zudem kann die Verwendung einer Reaktivverdünnermischung von Vorteil sein, um die Netzwerkdichte anzupassen und die Löslichkeit der ungesättigten Polyester im Reaktivverdünner zu erhöhen, die Bewitterungsbeständigkeit und Farbechtheit zu verbessern sowie um die Härtung zu beschleunigen oder anzupassen. Typische Reaktivverdünnermischungen enthalten neben Styrol insbesondere alpha-Methylstyrol, andere alkylierte Styrolderivate, Methylmethacrylat, tert.-Butylacrylat, Glycidylmethacrylat, Maleinsäureanhydrid, Trimethoxysilylpropylmethacrylat oder Triethoxysilylpropylmethacrylat. Grundsätzlich sind alle mit Styrol radikalisch copolymerisationsfähigen Monomere geeignet. Silane wie Trimethoxysilylpropylmethacrylat und Triethoxysilylpropylmethacrylat sind insbesondere dann ein günstiger Zusatz, wenn das erfindungsgemäße Harz als Beschichtung oder Imprägnierung von Glasfasern dienen soll, weil sie die Haftung des Duromers an Glasfasern verbessern.

Das ggf. mit Zusätzen versehene Harz (mit oder ohne Reaktivverdünner) kann als solches zu beliebig geformten Körpern oder in Form von auf einem Substrat befindlichen Beschichtungen ausgehärtet werden. In einer spezifischen Ausführungsform dient es zur Imprägnierung von textilen Flächengebilden wie Geweben, Gelegen oder dergleichen, deren Fasern beispielsweise aus Glas, Kohle, Aramid oder einem anderen Material bestehen können. Zur Herstellung der faserverstärkten Teile werden unter anderem das Laminierverfahren, das Faserspritzen, das Harzinjektionsverfahren (auch RTM genannt), das Wickelverfahren, das Pultrusionsverfahren, das Pressverfahren und das Spritzgussverfahren eingesetzt, und zwar jeweils in Kombination mit einem Imprägnierschritt. Zur Herstellung der unverstärkten Teile werden - nach einem vorangehenden Benetzungsschritt für Füllstoffe und Pigmente - vor allem das Gießverfahren sowie spezielle Imprägnierverfahren (zum Beispiel für Papier) eingesetzt. Beschichtungen werden unter anderem durch Tauchen, Spachteln, Sprühen, Streichen, Spritzen, Rakeln und Einspritzung aufgebracht.

Die Härtung der erfindungsgemäßen ungesättigten Polyesterharze zu den erfindungsgemäßen Werkstoffen kann gemäß dem Stand der Technik erfolgen. Dies umfasst vor allem die thermische (inkl. Raumtemperatur) und strahlenchemische Aushärtung. Dem Fachmann sind die hierfür erforderlichen thermischen und fotochemischen Initiatoren bekannt. In verschiedenen Fällen wird die Härtung ab Raumtemperatur angewandt, die kurz als Kalthärtung bezeichnet wird. Die Kalthärtung erfordert in der Regel Kombinationen von thermischen Initiatoren und sogenannten Beschleunigern, die dem Fachmann ebenfalls bekannt sind.

Vor der Härtung können dem Harz bei Bedarf Zusätze beigemischt werden. Falls ein Reaktivverdünner vorhanden ist, geschieht dies vorzugsweise nach dem Lösen der Harzkomponenten im Reaktivverdünner.

Den oder die Zusätze kann der Fachmann nach Bedarf auswählen. Beispielsweise können alle bekannten Zähmodifikatoren organischer und anorganischer Natur (z.B. Core-Shell-Partikel, Kautschuke, Wachse oder Silikone) additiv den neuen ungesättigten Polyesterharzen zugesetzt werden, um die Zähigkeit der resultierenden neuen Werkstoffe weiter zu erhöhen. Hierfür eignen sich in vielen Fällen auch die bekannten anorganischen Füllstoffe, wie zum Beispiel Kreide, Aluminiumhydroxid (ATH), Schwerspat, Quarzmehl, Kieselsäure. Günstig ist der Zusatz in Mengen kleiner 35 Gewichtsteile, bezogen auf 100 Gewichtsteile ungesättigtes Polyesterharz.

Darüber hinaus können bei der Verarbeitung der neuen erfindungsgemäßen ungesättigten Polyesterharze die dem Fachmann bekannten Additive zugesetzt werden, um die Verarbeitungs- und Werkstoffeigenschaften zu optimieren. Dazu zählen zum Beispiel Hautbildner zur Verringerung der Styrolemission bei der Verarbeitung, Prozessadditive zur Benetzung und Entlüftung, Thixotropieadditive (zum Beispiel disperse Kieselsäuren), weitere Inhibitoren zur Verlängerung der Lagerzeit von Harz und Harzmischung, Lichtschutzmittel (zum Beispiel UV-Absorber), Fasern, Füllstoffe, Farbstoffe, Pigmente und vieles andere mehr.

Die vorliegende Erfindung eignet sich insbesondere für Anwendungen, in denen das erfindungsgemäße Harz als Beschichtung oder in faserverstärkter Form eingesetzt werden soll. Gerade im Falle von Anwendungen in der Bauindustrie und im Fahrzeugbau sind beispielsweise häufig Schlag- und Splitterfestigkeit gefordert, und diese Anforderungen kann die vorliegende Erfindung erfüllen. Auch werden für Anwendungen im Außenbereich häufig die erfindungsgemäß erreichbaren Glastemperaturen benötigt (ca. 80 bis 100°C; die Erfindung erreicht 90 bis 110°C). Besonders bevorzugte Verwendungen für die Erfindung sind daher Windkraftanlagenflügel und -gehäuse, Bootswände und -aufbauten, Behälter wie Tanks, Rohrelemente, Platten, Pressteile für innen und außen, Schaltkästen, Bauelemente für den Hausbau sowie Teile für die Transportindustrie (z.B. zur Ausstattung von Eisenbahnwaggons) sowie den Automobilbereich (z.B. Karosserieteile, Scheinwerferreflektoren).

Die nachstehenden Beispiele dienen dem näheren Verständnis der Erfindung, ohne diese einzuschränken.

Harzbeispiel 1:

Ein ungesättigter Polyester wurde in einer Polykondensationsreaktion hergestellt. In einen 1-Liter Vier-Hals-Rundkolben wurden 1,36 mol Maleinsäureanhydrid, 0,41 mol Polypropylenglykol (Molmasse 425 g/mol), 0,95 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 18,43 mg KOH/g und einer Schmelzviskosität von 436 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 2:

Ein ungesättigter Polyester wurde in einer Polykondensationsreaktion hergestellt. In einen 1-Liter Vier-Hals-Rundkolben wurden 1,35 mol Maleinsäureanhydrid, 0,47 mol Polypropylenglykol (Molmasse 425 g/mol), 0,88 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 23,81 mg KOH/g und einer Schmelzviskosität von 238 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 3:

Ein ungesättigter Polyester wurde in einer Polykondensationsreaktion hergestellt. In einen 2-Liter Vier-Hals-Rundkolben wurden 2,69 mol Maleinsäureanhydrid, 1,08 mol Polypropylenglykol (Molmasse 425 g/mol), 1,61 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 22,80 mg KOH/g und einer Schmelzviskosität von 553 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 4:

Ein ungesättigter Polyester wurde in einer Polykondensationsreaktion hergestellt. In einen 1-Liter Vier-Hals-Rundkolben wurden 1,33 mol Maleinsäureanhydrid, 0,60 mol Polypropylenglykol (Molmasse 425 g/mol), 0,73 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 24,50 mg KOH/g und einer Schmelzviskosität von 122 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 5:

Ein ungesättigter Polyester wurde in einer Polykondensationsreaktion hergestellt. In einen 2-Liter Vier-Hals-Rundkolben wurden 2,76 mol Maleinsäureanhydrid, 1,38 mol Polypropylenglykol (Molmasse 425 g/mol), 1,38 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 26,09 mg KOH/g und einer Schmelzviskosität von 366 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 6:

Ein ungesättigter Polyester wurde in einer Polykondensationsreaktion hergestellt. In einen 2-Liter Vier-Hals-Rundkolben wurden 2,64 mol Maleinsäureanhydrid, 1,45 mol Polypropylenglykol (Molmasse 425 g/mol), 1,19 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 19,00 mg KOH/g und einer Schmelzviskosität von 346 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

Harzbeispiel 7:

Ein ungesättigter Polyester wurde in einer Polykondensationsreaktion hergestellt. In einen 2-Liter Vier-Hals-Rundkolben wurden 2,62 mol Maleinsäureanhydrid, 1,57 mol Polypropylenglykol (Molmasse 425 g/mol), 1,05 mol bispropoxyliertes Bisphenol A und 150 ppm Hydrochinon eingewogen und thermisch einer Polykondensationsreaktion unterzogen. Die Reaktion erfolgte unter Ausschluss von Luftsauerstoff. Das entstehende Reaktionswasser wurde mittels Destillation abgetrennt. Die Polykondensation wurde bis zu einer Säurezahl von 18,42 mg KOH/g und einer Schmelzviskosität von 112 mPas (150°C und 10000 1/s) geführt.

Das Harz wurde in 30 Massen-% Styrol gelöst. Die Harzlösung wurde mit 1,5 Massen-% Tert.-butylperethylhexanoat für eine Stunde bei 80°C und zwei Stunden bei 120°C gehärtet.

Die Bestimmung der Glasübergangstemperatur wurde mittel dynamisch-mechanischer Analyse (DMA) und die Bruchzähigkeit mittels Optical-Crack-Tracing (OCT) bestimmt (siehe Tabelle 1).

**Tabelle 1: Bruchzähigkeit und Glasübergangstemperaturen der gehärteten Harzbeispiele**

| Harzbeispiel | Bruchzähigkeit $K_{1C}$ [MN/m$^{3/2}$] | Glasübergangstemperatur [°C] |
|---|---|---|
| 1 | 0,397 | 110 |
| 2 | 0,733 | 99 |

(fortgesetzt)

| Harzbeispiel | Bruchzähigkeit $K_{1C}$ [MN/m$^{3/2}$] | Glasübergangstemperatur [°C] |
|---|---|---|
| 3 | 0,825 | 110 |
| 4 | 0,876 | 91 |
| 5 | 0,786 | 102 |
| 6 | 0,855 | 94 |
| 7 | 0,95 | 70 |

Es ist anzumerken, dass die vorgelegten Beispiele die Erreichbarkeit einer hohen Bruchzähigkeit in Kombination mit günstigen Glasübergangstemperaturen zeigen, ohne dass hierfür Ausführungsformen benutzt wurden, die das zu erwartende Wirkungsoptimum widerspiegeln, weil keine Hart- und Weichdiolsegmente mit identischer oder fast identischer Kettenlänge eingesetzt wurden.

**Patentansprüche**

1. Ungesättigter Carbonsäureester, erhalten aus oder unter Verwendung der nachfolgend in Formel (I) definierten Ausgangsmaterialien:

$$A_{(0,9-1,2)} \ (B + C)_{(1,0)} \qquad (I)$$

worin die in Klammern gesetzten Zahlen das molare Mengenverhältnis von Ausgangsmaterial A zur Summe der Ausgangsmaterialien B und C angeben und
worin bedeuten:

A: eine ungesättigte Dicarbonsäure,
B: ein Material B, ausgewählt unter Verbindungen, in denen ein Anteil von mindestens 40% der Atome der Verbindung einer kettensteifen Gruppierung angehört,
C: ein Material C, ausgewählt unter Verbindungen mit mindestens zwei Hydroxygruppen und einer durchgehenden Kette zwischen jeweils zwei Hydroxygruppen, die eine Länge von 10 bis 25 Atomen aufweist, wobei das Material C ausgewählt ist unter Verbindungen, in denen der Anteil, der einer kettensteifen Gruppierung angehört, höchstens 25% der Atome der Verbindung ausmacht,

wobei die Anteile der kettensteifen Gruppierung jeweils auf die Anzahl der C-, N-, O- und S-Atome in der Verbindung mit Ausnahme der Sauerstoffatome der endständigen Hydroxygruppen bezogen sind,
worin das molare Verhältnis von B zu C zwischen 5:95 und 95:5 liegt, und
worin die Anzahl der Glieder der durchgehenden Kette zwischen den beiden Hydroxygruppen im Material B und die Anzahl der Glieder der durchgehenden Kette zwischen den beiden Hydroxygruppen im Material C im Verhältnis von zwischen 1:2 und 2:1 liegt.

2. Ungesättigter Carbonsäureester nach Anspruch 1, worin das Material C nicht mehr als zwei Hydroxygruppen aufweist und worin das Material B ausgewählt ist unter Verbindungen mit zwei Hydroxygruppen und einer durchgehenden Kette zwischen den beiden Hydroxygruppen, die eine Länge von 5 bis 30 Atomen aufweist.

3. Ungesättigter Carbonsäureester nach Anspruch 1 oder 2, worin die Länge der durchgehenden Kette zwischen zwei Hydroxygruppen im Material B und/oder im Material C 10 bis 24 Atome beträgt.

4. Ungesättigter Carbonsäureester nach einem der Ansprüche 2 und 3, worin die Anzahl der Glieder der durchgehenden Kette zwischen den beiden Hydroxygruppen im Hartdiolsegment und die Anzahl der Glieder der durchgehenden Kette zwischen den beiden Hydroxygruppen im Weichdiolsegment im Verhältnis von zwischen 1:1,7 bis 1,7:1 liegt.

5. Ungesättigter Carbonsäureester nach einem der Ansprüche 2 bis 4, worin im Material B ein Anteil von mindestens 50%, vorzugsweise von mindestens 75% der Atome der Verbindung einer kettensteifen Gruppierung angehören, und/oder worin im Material C weniger als 15% der Atome einer kettensteifen Gruppierung angehören und vorzugsweise gar keine kettensteifen Gruppierungen vorhanden sind.

6. Ungesättiger Carbonsäureester nach einem der voranstehenden Ansprüche, worin das Material B ausgewählt ist unter dialkoxylierten Bisphenol A-, Bisphenol F- und Bisphenoltrimethylcyclohexan-Körpern.

7. Ungesättigter Carbonsäureester nach Anspruch 6, worin das Material C ausgewählt ist unter Polypropylenglykolen, Polypropylen-Polyethylencoglykolen, Polytetrahydrofuranen, Polymeren des 6-Caprolactons, 1,6-Hexandiolcarbonaten, $CO_2$-basierten Polycarbonaten sowie $\alpha,\omega$-hydroxyfunktionellen Verbindungen mit einer Kettenlänge von 10-25 Atomen, bei denen es sich um Kohlenstoffatome, gegebenenfalls unterbrochen durch O, S, NH oder NR, handelt.

8. Ungesättigtes Polyesterharz, umfassend
einen ungesättigten Carbonsäureester nach einem der Ansprüche 1 bis 7, gelöst in mindestens einer Verbindung mit mindestens einer C=C-Doppelbindung und ggf. einem aromatischen Ring, einem cycloaliphatischen Ring, einem Hetero-Ring, die/der unter dem Einfluss von Wärme, Licht oder ionisierender Strahlung in ein Kettenpolymer übergehen kann.

9. Ungesättigtes Polyesterharz nach Anspruch 8, weiterhin umfassend einen oder mehrere Zusätze, vorzugsweise ausgewählt unter Zähmodifikatoren, Verstärkungsfasern, Füllstoffen, Pigmenten, Farbstoffen und Prozessadditiven.

10. Ungesättigtes Polyesterharz nach Anspruch 9, worin der oder die Zähmodifikatoren ausgewählt sind unter Core-Shell-Partikeln, Kautschuken, Wachsen und Silikonen.

11. Formkörper oder Beschichtung, bestehend aus oder umfassend ein Duromer, das durch Härtung des Polyesterharzes nach einem der Ansprüche 8 bis 10 erhalten wurde.

12. Formkörper, umfassend ein Flächentextil, das mit einem Duromer beschichtet, getränkt, imprägniert oder laminiert ist, wobei das Duromer durch Härtung des Polyesterharzes nach einem der Ansprüche 8 bis 10 erhalten wurde.

## Claims

1. An unsaturated carboxylic acid ester obtained from or through the use of source or starting materials defined in formula (I):

$$A_{(0.9-1.2)} (B + C)_{(1.0)} \qquad (I)$$

wherein the figures in parentheses indicate the molar proportion of source material A to the sum of source materials B and C, and
wherein

A is an unsaturated dicarboxylic acid,
B is a material selected from compounds in which a proportion of at least 40% of the atoms belong to a chain-rigid group, and
C is a material selected from compounds comprising at least two hydroxyl groups and having a continuous chain between two respective hydroxyl groups, said chain having a length of 10 to 25 atoms, wherein the material C is selected from compounds in which the proportion belonging to a chain-rigid group accounts for not more than 25% of the atoms of the compound,

wherein the proportions of the chain-rigid group are respectively related to the number of C atoms, N atoms, O atoms and S atoms in the compound with the exception of the oxygen atoms of the terminal hydroxyl groups;
wherein the molar ratio of B to C is between 5:95 and 95:5; and
wherein the number of members of said continuous chain between both hydroxyl groups in said material B and the number of members of said continuous chain between both hydroxyl groups in said material C has a ratio of between 1:2 and 2:1.

**2.** An unsaturated carboxylic acid ester according to claim 1, wherein said material C comprises not more than two hydroxyl groups and wherein said material B is selected from compounds comprising two hydroxyl groups and having a continuous chain between both hydroxyl groups, which has a length of 5 to 30 atoms.

**3.** An unsaturated carboxylic acid ester according to claim 1 or 2, wherein the length of the continuous chain between two hydroxyl groups in said material B and/or in said material C is 10 to 24 atoms.

**4.** An unsaturated carboxylic acid ester according to any one of claim 2 or 3, wherein the number of members of said continuous chain between both hydroxyl groups in the hard diol segment and the number of members of the continuous chain between both hydroxyl groups in the soft diol segment has a ratio of between 1:1.7 to 1.7:1.

**5.** An unsaturated carboxylic acid ester according to any one of claims 2 to 4, wherein in the material B, a proportion of at least 50%, preferably at least 75%, of the atoms of the compound belong to a chain-rigid group, and/or wherein in the material C, less than 15% of the atoms belong to a chain-rigid group and preferably no chain-rigid groups are present.

**6.** An unsaturated carboxylic acid ester according to any one of the preceding claims, wherein said material B is selected from dialkoxylated bisphenol A bodies, bisphenol F bodies and bisphenol trimethylcyclohexane bodies.

**7.** An unsaturated carboxylic acid ester according to claim 6, wherein said material C is selected from polypropylene glycols, polypropylene/polyethylene co-glycols, polytetrahydrofuranes, polymers of 6-caprolactone, 1,6-hexandiol-carbonates, $CO_2$-based polycarbonates as well as $\alpha,\omega$-hydroxy functional compounds having a chain length of 10-25 atoms which are carbon atoms, optionally interrupted by O, S, NH or NR.

**8.** An unsaturated polyester resin, comprising
an unsaturated carboxylic acid ester according to any one of claims 1 to 7, which is dissolved in at least one compound having at least one C=C double bond and optionally an aromatic ring, a cycloaliphatic ring, a hetero ring, which can be converted to a chain polymer when exposed to heat, light and/or ionizing radiation.

**9.** An unsaturated polyester resin according to claim 8, further comprising one or more additives which are preferably selected from toughness modifiers, reinforcing fibers, fillers, pigments, dyes, and process additives.

**10.** An unsaturated polyester resin according to claim 9, wherein the toughness modifiers are selected from core-shell particles, rubbers, waxes, and silicones.

**11.** A molded article or a coating consisting of or comprising a duromer which has been obtained by curing the polyester resin according to any one of claims 8 to 10.

**12.** A molded article, comprising a two-dimensional fabric which is coated, soaked, impregnated or laminated with a duromer, wherein said duromer has been obtained by curing the polyester resin according to any one of claims 8 to 10.

**Revendications**

**1.** Ester d'acide carboxylique insaturé, obtenu à partir de ou par utilisation des matières premières définies ci-dessous dans la formule (I):

$$A_{(0,9-1,2)}(B+C)_{(1,0)} \qquad (I),$$

dans laquelle les chiffres placés entre parenthèses indiquent le rapport quantitatif molaire de la matière première A à la somme des matières premières B et C, et dans laquelle

A: représente un acide dicarboxylique insaturé,
B: représente un matériau B, sélectionné parmi des composés dans lesquels une part d'au moins 40 % des atomes du composé appartient à un groupe à chaîne rigide,
C: représente un matériau C, sélectionné parmi des composés avec au moins deux groupes hydroxy et entre chaque fois deux groupes hydroxy, une chaîne continue qui présente une longueur de 10 à 25 atomes, dans lequel le matériau C est sélectionné parmi des composés, dans lesquels la part, qui appartient à un groupe à chaîne rigide, constitue au plus 25 % des atomes du composé,

dans lequel les parts du groupe à chaîne rigide sont rapportées respectivement au nombre des atomes C, N, O et S dans le composé à l'exception des atomes d'oxygène des groupes hydroxy d'extrémité,
dans lequel le rapport molaire de B à C se situe entre 5:95 et 95:5, et
dans lequel le nombre des maillons de la chaîne continue entre les deux groupes hydroxy dans le matériau B et le nombre des maillons de la chaîne continue entre les deux groupes hydroxy dans le matériau C se situe dans un rapport compris entre 1:2 et 2:1.

2.  Ester d'acide carboxylique insaturé selon la revendication 1, dans lequel le matériau C ne présente pas plus de deux groupes hydroxy et dans lequel le matériau B est sélectionné parmi des composés avec deux groupes hydroxy et entre les deux groupes hydroxy, une chaîne continue qui présente une longueur de 5 à 30 atomes.

3.  Ester d'acide carboxylique insaturé selon la revendication 1 ou 2, dans lequel la longueur de la chaîne continue entre deux groupes hydroxy dans le matériau B et/ou dans le matériau C vaut 10 à 24 atomes.

4.  Ester d'acide carboxylique insaturé selon une des revendications 2 et 3, dans lequel le nombre des maillons de la chaîne continue entre les deux groupes hydroxy dans le segment de diol dur et le nombre des maillons de la chaîne continue entre les deux groupes hydroxy dans le segment de diol mou se situe dans le rapport compris entre 1:1,7 et 1,7:1.

5.  Ester d'acide carboxylique insaturé selon l'une quelconque des revendications 2 à 4, dans lequel dans le matériau B une part d'au moins 50 %, de préférence d'au moins 75 % des atomes du composé appartiennent à un groupe à chaîne rigide, et/ou dans lequel dans le matériau C moins de 15 % des atomes appartiennent à un groupe à chaîne rigide et de préférence même il ne se trouve aucun groupe à chaîne rigide.

6.  Ester d'acide carboxylique insaturé selon l'une quelconque des revendications précédentes, dans lequel le matériau B est sélectionné parmi des corps de bisphénol A, bisphénol F et bisphénol-triméthyl-cyclohexane dialcoxylés.

7.  Ester d'acide carboxylique insaturé selon la revendication 6, dans lequel le matériau C est sélectionné parmi les polypropylène glycols, les co-glycols de polypropylène-polyéthylène, les polytétrahydrofuranes, les polymères du 6-caprolactone, les carbonates de 1,6-hexanediol, les polycarbonates à base de $CO_2$ ainsi que les composés hydroxyfonctionnels $\alpha$, $\omega$ avec une longueur de chaîne de 10-25 atomes, dans lesquels il s'agit d'atomes de carbone, éventuellement interrompus par O, S, NH ou NR.

8.  Résine de polyester insaturée, comprenant un ester d'acide carboxylique insaturé selon l'une quelconque des revendications 1 à 7, dissous dans au moins un composé avec au moins une double liaison C=C et éventuellement un anneau aromatique, un anneau cycloaliphatique, un hétéro-anneau, qui peut se transformer en une chaîne polymère sous l'influence de la chaleur, de la lumière ou d'un rayonnement ionisant.

9.  Résine de polyester insaturée selon la revendication 8, comprenant en outre un ou plusieurs additif(s), de préférence sélectionné(s) parmi les modificateurs de ténacité, les fibres de renforcement, les matières de charge, les pigments, les colorants et les additifs de traitement.

10. Résine de polyester insaturée selon la revendication 9, dans laquelle le ou les modificateur(s) de ténacité est/sont sélectionné(s) parmi les particules Core-Shell, les caoutchoucs, les cires et les silicones.

11. Corps moulé ou revêtement, composé de ou comprenant un duromère, qui a été obtenu par durcissement de la résine de polyester selon l'une quelconque des revendications 8 à 10.

12. Corps moulé, comprenant un textile plat, qui est revêtu, imbibé, imprégné ou stratifié avec un duromère, dans lequel le duromère a été obtenu par durcissement de la résine de polyester selon l'une quelconque des revendications 8 à 10.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69924863 T2
- DE 4406646 A1

- GB 722265 A

**In der Beschreibung aufgeführte Nicht-Patentliteratur**

- **S. B. PANDIT ; V. M. NADKARNI.** Toughening of unsaturated Polyesters by Reactive Liquid Polymers. 1. Synthesis and Characterization of the Modifiers. *Ind. Eng. Chem. Res.,* 1993, vol. 32, 3089-3099

- Toughening of unsaturated Polyesters by Reactive Liquid Polymers. 2. Processibility and mechanical Properties. *Ind. Eng. Chem. Res,* 1994, vol. 33, 2778-2788
- **MIKHAIOLOVA, Z.V. et al.** *In Polimery,* 1979, vol. 24 (11-12), 407-409
- **M. BALLAUF.** *Angewandte Chemie,* 1989, vol. 101 (3), 362-410